# EUROPEAN PATENT SPECIFICATION

(11) **EP 0 757 056 B1**
(45) Date of publication and mention of the grant of the patent: **11.11.1998**
(21) Application number: 96111996.3
(22) Date of filing: 25.07.1996
(51) Int. Cl.: C07H 19/09

(54) **Method for preparing 1-beta-D-arabinofuranosylcytosine**
Verfahren zur Herstellung von 1-Beta-D-Arabinofuranosylcytosine
Méthode pour préparer 1-bêta-D-arabinofuranosylcytosine

(30) Priority: 03.08.1995 IT MI951715
(43) Date of publication of application: 05.02.1997
(73) Proprietor: PRO.BIO.SINT. S.r.l., 21100 Varese (IT)
(72) Inventor: De Meglio, Giuseppe, 20129 Milano (IT); Ordanini, Giancarlo, 21039 Bedero Valcuvia, Varese (IT); Bruzzese, Tiberio, 20146 Milano (IT)
(74) Representative: Luksch, Giorgio, Dr.-Ing.

(56) References cited:
- WO-A-91/13900
- DE-A- 2 146 733

## Description

The present invention refers to a new synthetic process for preparing the nucleosidic derivative: 1-β-D-arabinofuranosylcytosine also known as cytarabine or Ara-C (I).

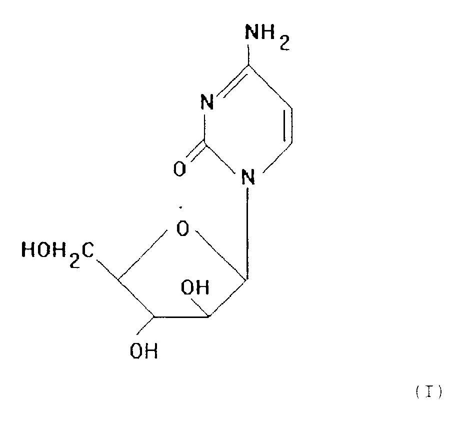

Ara-C is a known compound useful as an antiviral drug, highly specific for curing some tumours such as leukaemia.

Ara-C is prepared by a glycosylation reaction between silylated cytosine (II) (or silylated 1-N-acetylcytosine) and an arabinose derivative (III) and then hydrolysing the condensation product (US 3.721.664 and US 3.868.373) as illustrated in the following reaction scheme:

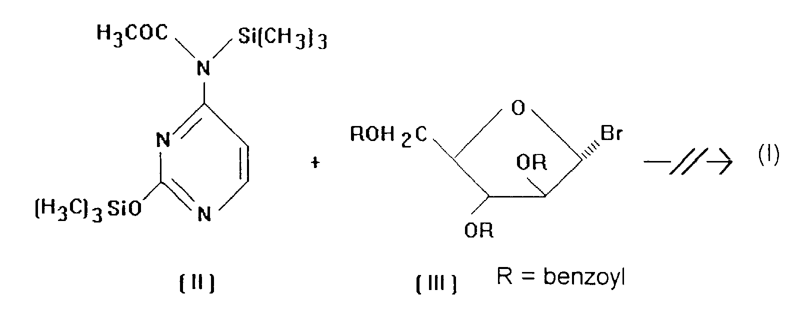

It has been found that the synthesis is extremely toilsome since it requires the preparation of an arabinose derivative and carries to a mixture of the two protected isomers of Ara-C (α + β) which, after separation, are hydrolysed.

Alternatively, Ara-C is prepared by constructing the pyrimidinic ring on the arabinose molecule (DE 2.027.305 and DE 2.601.399) according to the following reaction scheme:

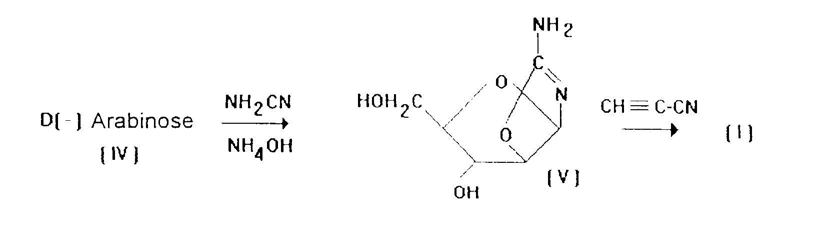

Also such synthesis was found to be toilsome since it requires several steps and uses dangerous reactants thus resulting in being inapplicable on an industrial scale.

Still another synthesis of Ara-C provides its preparation by ammoniacal hydrolysis of derivatives of the cyclocytidine (VII) obtained from cytidine (VI) according to the following reaction scheme:

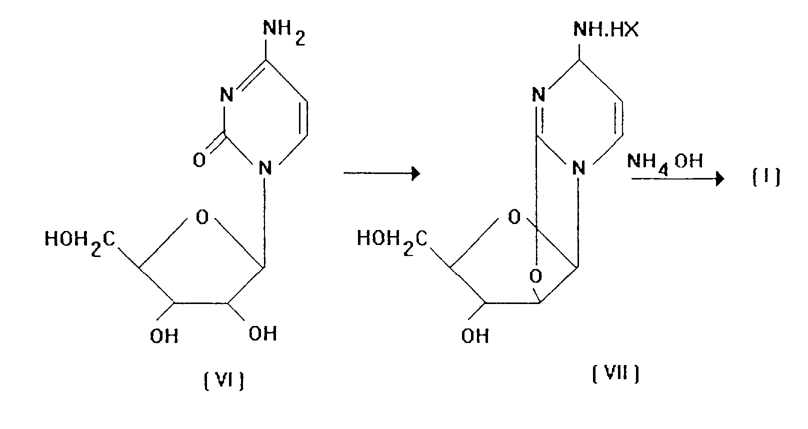

Even if a number of methods for preparing cytidine (even lately: PCT Int. Appl. WO 91 13900, wherein cytidine (VI) is treated first with dibutyl tin oxide and then with triethylamine and p-toluene sulfochloride) were disclosed, wherein said intermediate was obtained with acceptable yields, an obstacle to such synthesis, from the point of view of the industrial realisation, derives from the fact that cytidine was used, which is a product which allows several industrial uses (amongst others, it is useful to synthesise cytidine-choline diphosphate, namely cytilcholine), having a quite high price and of limited availability. Other syntheses of Ara-C introduce, in the position 4 of the pyrimidinic ring of a protected derivative of Ara-U (VIII), particular chemical groups which can give rise to an easy aminolysis reaction:

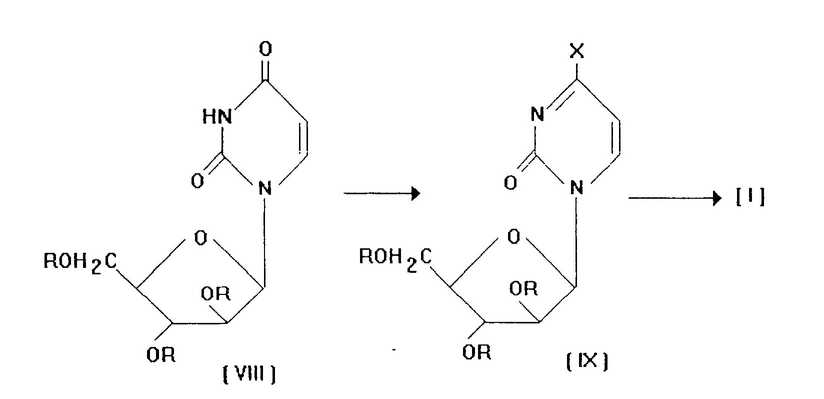

Among these syntheses there are contemplated introducing a thio group (X = SH) by heat reacting the compound (VIII) (R = acetyl) with phosphorous pentasulfide in pyridine and subsequent aminolysis in an autoclave, under heating, with methanol and ammonia gas (US 3.116.282); introducing a chlorine atom (X = Cl) by heat reacting the compound (VIII) (R = benzoyl) with phosphorous oxychloride and triethylamine hydrochloride in ethyl acetate and subsequent treatment with ammonia gas and final hydrolysis (DE 2.146.733); introducing an imidazole group 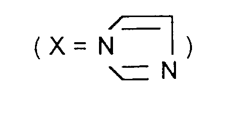 by heat reacting the compound (VIII) (R = acetyl) with (C₆H₅O)₂POCl, triethylamine and imidazole and subsequent aminolysis with aqueous ammonia (JP Kokai JP-01-143892); introducing a sulfonyl group (X = -OSO₂R) by heat reacting of the compound (VIII) (R = acetyl) with conc. p-toluensulfochloride and potassium carbonate in dichloroethane and subsequent aminolysis with ammonia gas and final hydrolysis (EP-A-0204264).

All these synthetic methods are toilsome, they require several steps and carry to yields which are relatively low. Besides, the intermediate compounds (IX) must often be treated with a particular care because of their low stability.

The present invention describes a process for preparing 1-β-D-arabinofuranosylcytosine (Ara-C) (I), according to the following scheme: 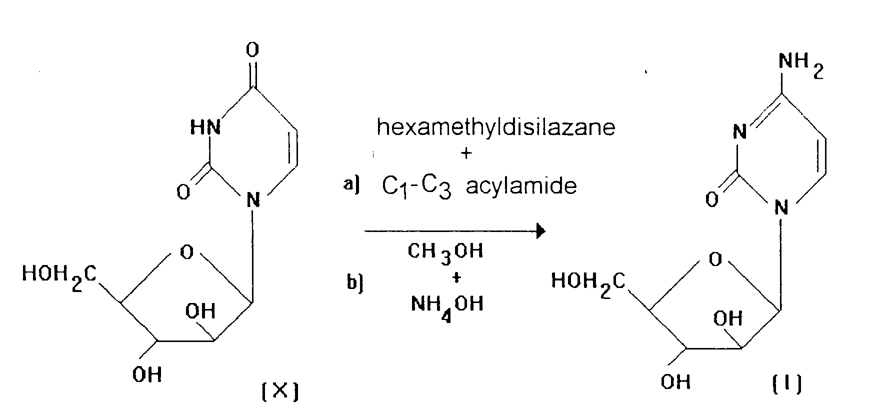 which comprises the reaction of 1-β-D-arabinofuranosyluracil [Ara-U (X)], under a pressure between 8 and 18 bar and at a temperature between 130 and 140°C, with hexamethyldisilazane and a C₁-C₃ acylamide, subsequently dry evaporating the resulting mixture and subsequently treating the oily residual thereby obtained with methyl alcohol and aqueous ammonia, further dry evaporating and purifying said residual by percolating on an acidic resin, washing with water and subsequently eluting with ammonia, finally isolating Ara-C by procedures common in the field.

The process of the present application allows to obtain Ara-C directly from unprotected Ara-U (X) in a single and easy step. The starting material, Ara-U, is prepared very easily from uridine (XI), a commercial material widely available and having a low price, according to the following reactions, well known in literature:

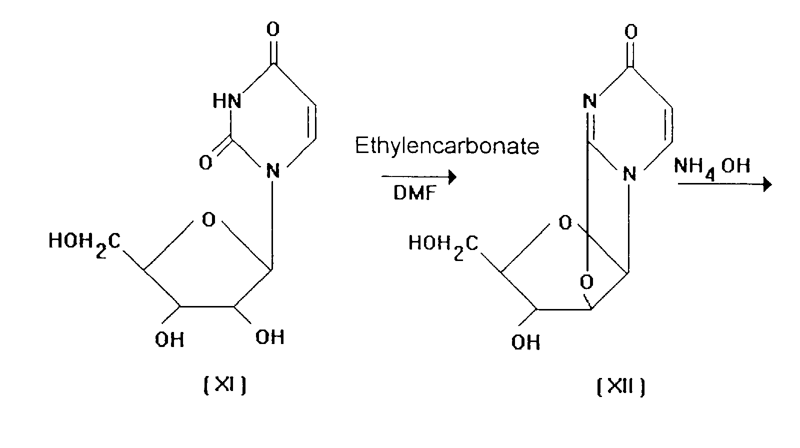

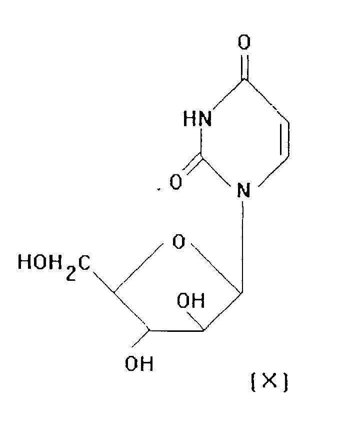

According to the process of the invention, Ara-U is treated in autoclave, under controlled pressure and temperature, with hexamethyldisilazane and a C₁-C₃ acylamide, preferably acetamide, obtaining trimethylsilylated Ara-U first and then aminating in position 4.

After cooling, evaporating the solvent and treatment, at room temperature, the residual obtained with methanol and aqueous ammonia, to eliminate the protecting groups, the mixture is evaporated under vacuum again. The new residual is dissolved in water and percolated on an acidic resin. The resin is first washed with water until total elimination of unreacted Ara-U, then eluted with 2.5% ammonia. The ammoniacal solution is evaporated under vacuum, stripping the oily residual with methanol and dissolving in methanol and filtering the solid obtained.

In the present invention the expression "acidic resin" denotes a ion exchange resin with several matrixes, particularly polystyrenic ones, and having an active group preferably consisting of a sulfonic radical. Amberlites, such as the IR 120, Dowexes, Relites, etc., belong to this groups of resins.

Preferably the heating step in autoclave is prolonged for 1-3 days under a pressure from 10 to 16 bar and at a temperature of about 135°C. It is preferred to use 1.5-3 mol of acetamide per mole of Ara-U, particularly 2.5 mol of acetamide, whereas in the case of hexamethyldisilazane it is preferred to use an amount of about 7-10 mol per mole of Ara-U, particularly 9 mol of hexamethyldisilazane.

The process of the present invention has several advantages since it allows a simple carrying out of the synthetic process, directly using Ara-U and therefore rendering superfluous the preparation of its triacetylated or tribenzoylated derivatives; the process of the invention further allows the reduction of the costs since the reactants have low prices, carries to high yields of 75-85% compared to the theoretical value of the product and allows to obtain a product having a high purity and an easy recovery of unreacted Ara-U.

The following examples illustrate the invention without limiting it.

### Example 1

In a 500 ml autoclave, 24.42 g (0,1 mol) of Ara-U, 187.5 ml (0.9 mol) of hexamethyldisilazane and 14.76 g (0.250 mol) of acetamide, are charged.

The mixture is heated at 135°C under stirring for 45 hours, under a pressure of about 15 bar. After cooling, the autoclave is slowly opened and, after having added 60 ml of methanol, the solution is evaporated under vacuum. The oily residual obtained is then treated at room temperature for 90 minutes with 300 ml of methanol and 70 ml of 25% aqueous ammonia. The solution (a HPLC analysis shows 83.22% of Ara-C and 14.6% of Ara-U) is then evaporated, stripping the residual with methanol to fully eliminate ammonia, then it is dissolved in 800 ml of water and percolated on 300 g of resin CFS(H⁺) (cationic, strong, sulfonic, in acidic phase). The resin is washed with water till 40 mg/l of Ara-U are obtained, then it is eluted with 2.5% water ammonia. After evaporating of the ammoniacal waters, the oily product is crystallised by some strippings with methanol, then it is dissolved in 50 ml of methanol. After cooling, the precipitate was filtered and dried.
Yield = 18.75 g (77.16% compared to the theoretical value)
m.p. = > 210°C
weight loss = 0.71%
TLC = CHCl₃/CH₃OH solvent - 50/50 - unitary spot
Tit. UV = 97.93%
HPLC = 99.86%
[α]²⁰ = +156°C (1% in H₂O)

### Example 2

Proceeding analogously to Example 1, the autoclave was heated for 72 h.

The HPLC analysis of the solution, after treatment with methyl alcohol and aqueous ammonia, showed 92.84% of Ara-C and 6.39% of Ara-U.

After purifying on resin, 20.15 g (82.9% compared to theoretical value) was obtained.

## Claims

1. A process for preparing 1-β-D-arabino furanosylcytosine having the following formula (I): 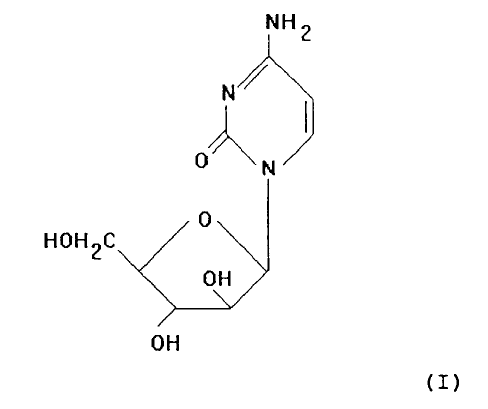 which comprises reacting 1-β-D-arabinofurano syluracil (X), under a pressure between 8 and 18 bar and at a temperature between 130 and 140°C, with hexamethyldisilazane and a C₁-C₃ acylamide, subsequently dry evaporating the resulting mixture and subsequently treating the oily residual thereby obtained with methyl alcohol and aqueous ammonia, further dry evaporating and purifying said residual by percolating on an acidic resin, washing with water and subsequently eluting with ammonia, finally isolating Ara-C (I) by procedures common in the field.

2. A process according to claim 1, wherein the pressure is between 10 and 16 bar and the temperature is maintained at 135°C for 1-3 days.

3. A process according to any one of the preceding claims, wherein the C₁-C₃ acylamide is acetamide.

4. A process according to any one of the preceding claims, wherein the molar ratio between hexamethyldisilazane and Ara-U is between 7 and 10.

5. A process according to any one of the preceding claims, wherein the molar ratio between hexamethyldisilazane and Ara-U is 9.

6. A process according to any one of the preceding claims, wherein the molar ratio between the C₁-C₃ acylamide and Ara-U is between 1.5 and 3.

7. A process according to any one of the preceding claims, wherein the molar ratio between C₁-C₃ acylamide and Ara-U is 2.5.

## Patentansprüche

1. Verfahren zur Herstellung von 1-β-D-Arabinofuranosylcytosin mit der folgenden Formel (I): 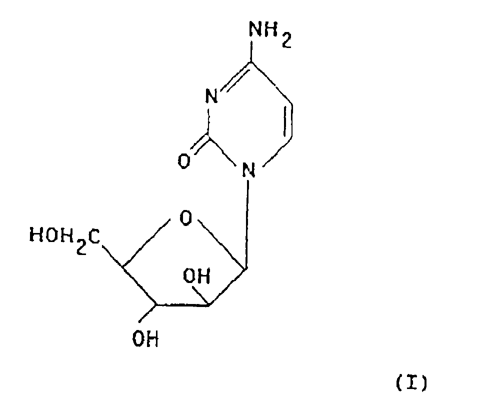 welches umfaßt: Umsetzen von 1-β-D-Arabinofuranosyluracil (X) unter einem Druck von zwischen 8 und 18 bar und bei einer Temperatur zwischen 130 und 140°C mit Hexamethyldisilazan und einem C₁₋₃-Acylamid, anschließend Eindampfen der resultierenden Mischung zur Trockne und anschließend Behandeln des dadurch erhaltenen öligen Rückstandes mit Methylalkohol und wäßrigem Ammoniak, ferner Eindampfen zur Trockne und Reinigen des Rückstandes durch Perkolieren an einem sauren Harz, Waschen mit Wasser und anschließend Eluieren mit Ammoniak, und schließlich Isolieren von Ara-C (I) durch auf diesem Gebiet übliche Verfahren.

2. Verfahren gemäß Anspruch 1, worin der Druck zwischen 10 und 16 bar beträgt und die Temperatur für 1 bis 3 Tage auf 135°C gehalten wird.

3. Verfahren gemäß einem der vorhergehenden Ansprüche, worin das C₁₋₃-Acylamid Acetamid ist.

4. Verfahren gemäß einem der vorhergehenden Ansprüche, worin das Molverhältnis zwischen Hexamethyldisilazan und Ara-U zwischen 7 und 10 beträgt.

5. Verfahren gemäß einem der vorhergehenden Ansprüche, worin das Molverhältnis zwischen Hexamethyldisilazan und Ara-U 9 beträgt.

6. Verfahren gemäß einem der vorhergehenden Ansprüche, worin das Molverhältnis zwischen dem C₁₋₃-Acylamid und Ara-U zwischen 1,5 und 3 beträgt.

7. Verfahren gemäß einem der vorhergehenden Ansprüche, worin das Molverhältnis zwischen dem C₁₋₃-Acylamid und Ara-U 2,5 beträgt.

## Revendications

1. Procédé de préparation de la 1-β-D-arabinofurannosylcytosine, ayant la formule (1) ci-après : 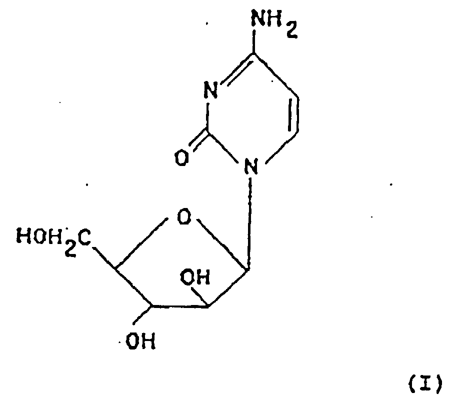 qui consiste à faire réagir du 1-β-D-arabinofurannosyluracile (X), sous une pression comprise entre 8 et 18 bar et à une température comprise entre 130 et 140°C, avec de l'hexaméthyldisilazane et un acylamide en C₁-C₃, puis à évaporer à sec le mélange obtenu, puis à traiter le résidu huileux ainsi obtenu avec de l'alcool méthylique et de l'ammoniaque, puis à évaporer à sec et à purifier ledit résidu par percolation sur une résine acide, à laver avec de l'eau, puis à éluer avec de l'ammoniac, et finalement à isoler l'Ara-C (I) pour des techniques connues.

2. Procédé selon la revendication 1, dans lequel la pression est comprise entre 10 et 16 bar, et la température est maintenue à 135°C pendant 1 à 3 jours.

3. Procédé selon l'une quelconque des revendications précédentes, dans lequel l'acylamide en C₁-C₃ est l'acétamide.

4. Procédé selon l'une quelconque des revendications précédentes, dans lequel le rapport en moles entre l'hexaméthyldisilazane et l'Ara-U est compris entre 7 et 10.

5. Procédé selon l'une quelconque des revendications précédentes, dans lequel le rapport en moles entre l'hexaméthyldisilazane et l'Ara-U est de 9.

6. Procédé selon l'une quelconque des revendications précédentes, dans lequel le rapport en moles entre l'acylamide en C₁-C₃ et l'Ara-U est compris entre 1,5 et 3.

7. Procédé selon l'une quelconque des revendications précédentes, dans lequel le rapport en moles entre l'acylamide en C₁-C₃ et l'Ara-U est de 2,5.
